# EUROPÄISCHE PATENTANMELDUNG

(11) **EP 2 210 558 A1**
(43) Veröffentlichungstag der Anmeldung: **28.07.2010**
(21) Anmeldenummer: 09000748.5
(22) Anmeldetag: 21.01.2009
(51) Int. Cl.: A61B 5/151, A61M 5/32

(54) **Lanzette mit Kapillarkanal und Sterilschutz sowie Verfahren zur Herstellung einer solchen Lanzette**

(71) Anmelder: Roche Diagnostics GmbH, 68305 Mannheim (DE); F. Hoffmann-La Roche AG, 4070 Basel (CH)
(72) Erfinder: Harttig, Herbert, 67434 Neustadt (DE)
(74) Vertreter: Mommer, Niels

(57) **Zusammenfassung**

Die Erfindung betrifft eine Lanzette mit einem Stechelement (1) zum Einstich in die Haut eines Patienten, einem Testbereich (5) mit Nachweisreagenzien zur Bestimmung einer Analytkonzentration einer Körperflüssigkeitsprobe, einer in dem Stechelement (1) angeordneten Kapillarstruktur (3), um eine Körperflüssigkeitsprobe zu dem Testbereich (5) zu transportieren, und einem Sterilschutz (6), der die Spitze des Stechelements (4) umschließt. Erfindungsgemäß ist vorgesehen, dass die Kapillarstruktur (3) in einem von dem Sterilschutz (6) umgebenen Abschnitt blockiert ist. Die Erfindung betrifft ferner ein Verfahren zum Herstellen einer Lanzette mit den Folgenden Schritten: Herstellen eines Stechelements (1), das auf seiner Oberseite eine nach oben offene Kapillarstruktur (3) aufweist, Bedecken eines Abschnitts der Oberseite des Stechelements (1) und Blockieren des Kapillarkanals (3) in diesem Abschnitt mit einem ersten Material, Bedecken eines Abschnitts der Unterseite mit einem zweiten Material, Sterilisieren des Stechelements (1), Anbringen eines Testbereichs (5) mit Nachweisreagenzien an dem Stechelement (1).

## Beschreibung

Die Erfindung geht aus von einer Lanzette mit den im Oberbegriff des Anspruchs 1 angegebenen Merkmalen, wie sie aus der EP 1360935 A1 bekannt ist. Die Erfindung betrifft ferner ein Verfahren zum Herstellen einer derartigen Lanzette mit einem Sterilschutz.

Aus der EP 1360935 A1 sind Lanzetten mit integrierten Testbereichen, die Nachweisreagenzien zur Bestimmung einer Analytkonzentration, beispielsweise der Glucosekonzentration enthalten, bekannt. Die Lanzetten sind einzelnen in Kammern eines Trägerbandes versiegelt und so vor schädlichen Umwelteinflüssen geschützt. Allerdings besteht bei der Sterilisation der Lanzetten die Gefahr, dass die Testbereiche beeinträchtigt werden.

Aufgabe der Erfindung ist es deshalb, einen Weg aufzuzeigen, wie sich kostengünstig Lanzetten herstellen und lagern lassen, die einem Testbereich mit Nachweisreagenzien und eine Kapillarstruktur zum Transport einer Körperflüssigkeitsprobe zu dem Testbereich aufweisen.

Diese Aufgabe wird durch eine verpackte Lanzette mit den im Anspruch 1 angegebenen Merkmalen sowie durch ein Verfahren mit den im Anspruch 14 angegebenen Merkmalen gelöst. Vorteilhafte Weiterbildungen der Erfindung sind Gegenstand der Unteransprüche.

Erfindungsgemäß ist vorgesehen, dass die Kapillarstruktur in einem von dem Sterilschutz umgebenen Bereich blockiert ist, bevorzugt indem der Sterilschutz die Kapillarstruktur zumindest auf einem Teil ihrer Länge ausfüllt.

Vorteilhaft lässt sich zur Herstellung einer erfindungsgemäßen Lanzette zunächst die Spitze des Stechelements in den Sterilschutz einschließen, so dass das Stechelement nach der Sterilisation problemlos ohne Gefahr einer Kontamination gehandhabt werden kann. Insbesondere lässt sich an dem sterilisierten Stechelement später ohne Schwierigkeiten ein Testbereich anbringen, beispielsweise indem ein Testbereichträger an dem Stechelement befestigt wird. Empfindliche Nachweisreagenzien des Testbereichs werden bei einer solchen Vorgehensweise von dem Sterilisationsvorgang nicht beeinträchtigt. Ein aufwändiges Abdecken des Testbereichs bei einer Sterilisation der Lanzette durch Strahlungseinwirkung ist dann selbst bei Verwendung strahlungsempfindlicher Nachweißreagenzien nicht erforderlich, da der Testbereich erst nach der Sterilisation an dem Stechelement angebracht wird, sich also auf einem aus dem Sterilschutz herausragenden Teil der Lanzette befindet.

Vorteilhaft können bei einer erfindungsgemäßen Lanzette die Spitze des Stechelements und der bei einem Stich in den Körper eines Patienten eindringende Teil der Kapillarstruktur selbst bei sehr langer Lagerung funktionsfähig, sauber und steril gehalten werden. Da die Kapillarstruktur blockiert ist, ist bei einer erfindungsgemäßen Lanzette ausgeschlossen, dass Material von dem Testbereich über die Kapillarstruktur in einen vorderen Teil der Lanzette und bei einem Stich in den Körper eines Patienten gelangt. Bei einem einfachen Versiegeln einer Lanzette in einer Kammer, wie es aus der EP 1 360 935 A1 bekannt ist, besteht dagegen die Gefahr, dass Enzyme oder Staub im Lauf der Zeit in die Kapillarstruktur wandern und dort im Laufe der Zeit eine hydrophobisierende Wirkung entfalten oder sogar bei einem Stich in den Körper des Patienten gelangen und dort Schaden verursachen.

Bevorzugt wird das Stechelement erst nach dem Anbringen des Sterilschutzes sterilisiert, bevorzugt durch Strahlungseinwirkung, beispielsweise Elektronen- oder Gammastrahlung. Prinzipiell ist es jedoch auch möglich, das Stechelement zuerst zu sterilisieren und den Sterilschutz anschließend anzubringen. Die entsprechenden Schritte b) bis d) des Anspruchs 14 können in beliebiger Reihenfolge durchgeführt werden. Die Reihenfolge der Aufzählung der Schritte in Anspruch 14 bedeutet nicht, dass die Schritte in dieser Reihenfolge durchgeführt werden müssen.

Bevorzugt wird eine erfindungsgemäße Lanzette auf einem Trägerband angeordnet. Besonders bevorzugt ist dabei insbesondere, die einzelnen Lanzetten mit dem Sterilschutz in Kammern zu versiegeln. Geeignete Kammern lassen sich durch ein Trägerband und eine die Lanzetten jeweils bedeckende Folie leicht herstellen.

Bevorzugt ist der Sterilschutz einer erfindungsgemäßen Lanzette aus mindestens zwei Komponenten zusammen gefügt. Dabei bedeckt eine erste Komponente einen Abschnitt einer Oberseite des Stechelements und füllt in dem bedeckten Abschnitt die Kapillarstruktur aus. Bevorzugt ist die erste Komponente eine Folie. Eine zweite Komponente bedeckt einen Abschnitt einer Unterseite des Stechelements, so dass dessen Spitze umschlossen ist. Bevorzugt ist die zweite Komponente eine Folie. Als Oberseite und Unterseite sind dabei zwei gegenüberliegende Bereiche des Stechelements anzusehen, die sich in dessen Längsrichtung erstrecken. Beispielsweise kann das Stechelement näherungsweise zylindrisch geformt sein, so dass die Oberseite und die Unterseite jeweils näherungsweise einen halbkreisförmigen Querschnitt aufweisen. Bevorzugt ist das Stechelement jedoch flach, beispielsweise aus Metallblech ausgeschnitten.

Beispielsweise kann die Kapillarstruktur mit der ersten Komponente ausgegossen sein. Bevorzugt ist die erste Komponente jedoch in die Kapillarstruktur eingepresst, da sich die erste Komponente dann leichter wieder aus der Kapillarstruktur entfernen lässt. Besondere bevorzugt wird die erste Komponente beim Einpressen plastisch verformt. Beispielsweise kann das Stechelement zwischen zwei Folien gelegt werden, die dann verpresst werden, so dass sich eine der beiden Folien als erste Komponente verformt und in die Kapillarstruktur gepresst wird. Die beiden Folien werden dann stoffschlüssig verbunden und können beispielsweise miteinander verklebt oder verschweißt sein. Bevorzugt ist die erste Folie dicker als die zweite Folie, vorzugsweise mindestens fünfmal so dick, insbesondere mindestens zehnmal so dick. Besonders bevorzugt ist die Dicke der ersten Folie größer als die Tiefe der Kapillarstruktur, insbesondere mindestens 10% größer. Besonders bevorzugt ist die zweite Folie dünner als das Stechelement und hat beispielsweise eine Dicke von nicht mehr als 20% der Dicke des Stechelements.

Die erste Komponente ist bevorzugt ein Polymerwerkstoff. Es können jedoch beispielsweise auch metallische Werkstoffe, beispielsweise Metallschäume oder Fasermaterial, verwendet werden.

Bevorzugt ist die erste Komponente porös, beispielsweise ein Schaumwerkstoff, insbesondere ein Polymerschaum. Diese Maßnahme hat den Vorteil, dass sich die erste Komponente besonders gut in die Kapillarstruktur pressen und wieder daraus entfernen lässt.

Die Kapillarstruktur einer erfindungsgemäßen Lanzette ist bevorzugt ein Kapillarkanal. Der Kapillarkanal ist bevorzugt als eine Rinne ausgebildet. Möglich ist es aber auch, dass der Kapillarkanal ein durchgehender Schlitz ist, so dass der Kapillarkanal sowohl zur Oberseite als auch zur Unterseite des Stechelements hin offen ist. Bevorzugt ist die Kapillarstruktur hydrophilisiert, beispielsweise durch eine Plasmabehandlung oder indem sie mit einem hydrophilen Überzug, beispielsweise aus Heparin, versehen ist.

Weitere Einzelheiten und Vorteile der Erfindung werden an einem Ausführungsbeispiel unter Bezugnahme auf die beigefügten Zeichnungen erläutert. Es zeigen:
- Figur 1: ein Ausführungsbeispiel einer erfindungsgemäßen Lanzette ohne Ste- rilschutz;
- Figur 2: derartige Lanzetten mit Sterilschutz auf einem Trägerband;
- Figur 3: einen Querschnitt durch eine Lanzette entlang der Schnittlinie AA in Figur 2;
- Figur 4: eine Seitenansicht zu Figur 2;
- Figur 5: das Entfernen des Sterilschutzes.

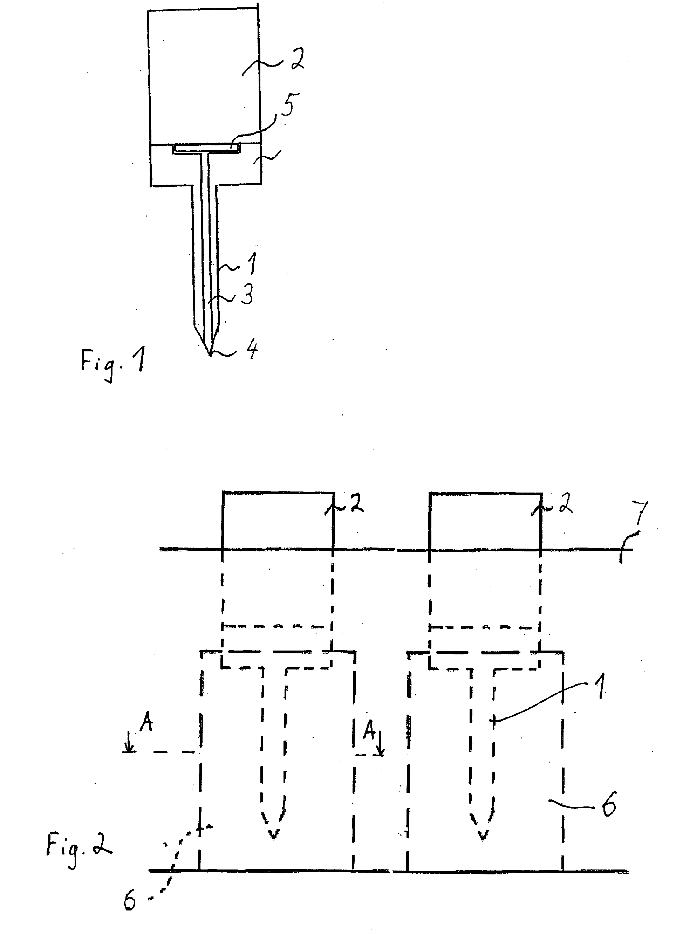

Die in Figur 1 schematisch dargestellte Lanzette hat ein Stechelement 1 und einen Trägerkörper 2. Das Stechelement 1 ist bevorzugt aus Metall, insbesondere aus Stahl, und kann beispielsweise aus Blech ausgeschnitten sein, beispielsweise durch Stanzen, Ätzen oder Laserstrahlschneiden. Das Stechelement 1 kann aber beispielsweise auch aus einem keramischen Material oder Kunststoff hergestellt sein. Ein geeignetes Stechelement kann beispielsweise auf die aus der WO 2006/066744 A1 beschriebene Weise hergestellt sein.

In dem Stechelement 1 ist als Kapillarstruktur ein Kapillarkanal 3 angeordnet, der bei dem dargestellten Ausführungsbeispiel von der Spitze 4 des Stechelements 1 ausgeht. Eine Kapillarstruktur ist eine derart bemessene Struktur, dass eine Körperflüssigkeitsprobe darin durch Kapillarkräfte bewegt wird. Dabei ist es auch möglich, den Kapillarkanal 3 erst in einem Abstand von der Spitze 4 des Stechelements 1 beginnen zu lassen. Wichtig ist in diesem Zusammenhang lediglich, dass der Kapillarkanal 3 bei einem Stich soweit in den Körper eines Patienten eindringt, dass Körperflüssigkeit in den Kapillarkanal 3 gelangen und dann durch Kapillarkräfte transportiert werden kann.

Der Kapillarkanal 3 mündet in einem Testbereich 5 mit Nachweisreagenzien zur Bestimmung einer Analytkonzentration einer Körperflüssigkeitsprobe. Der Testbereich 5 kann beispielsweise mit Elektroden zur elektrochemischen Konzentrationsbestimmung versehen sein oder durch eine konzentrationsabhängige Verfärbung der Nachweisreagenzien eine photometrische Konzentrationsbestimmung ermöglichen. Bei dem dargestellten Ausführungsbeispiel ist der Testbereich 5 auf dem Trägerkörper 2 angeordnet, der bevorzugt aus Kunststoff ist, jedoch auch aus anderen Materialen, beispielsweise Metall, Keramik oder Halbleitermaterial bestehen kann.

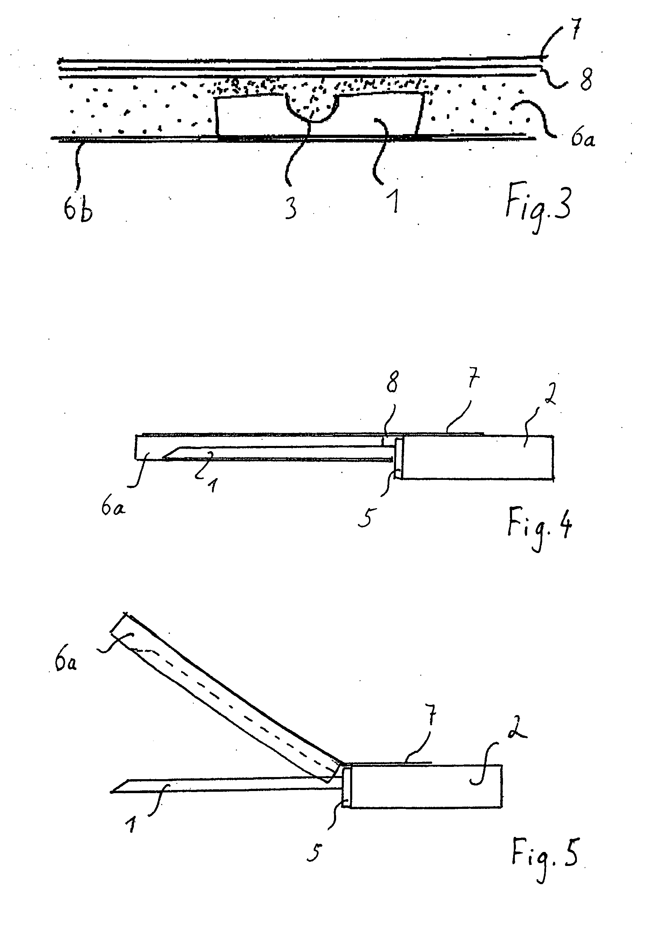

Figur 2 zeigt in einer schematischen Darstellung mehrere Lanzetten mit einem die Spitze 4 des Stechelements 1 umschließenden Sterilschutz 6 auf einem Trägerband 7. Figur 3 zeigt eine Schnittansicht entlang der Schnittlinie AA der Figur 2. Der von dem Sterilschutz 6a, 6b umschlossenen Abschnitt des Stechelements 1 hat eine Länge von bevorzugt mehr als 2mm, beispielsweise 2 bis 3 mm.

Wie Figur 2 zeigt, ragt das Stechelement 1 aus dem Sterilschutz 6 heraus. Der Trägerkörper 2 und der Testbereich 5 sind außerhalb des Sterilschutzes 6 angeordnet. Der Sterilschutz 6 ist aus zwei Komponenten 6a, 6b zusammengefügt, die schematisch in Figur 3 dargestellt sind. Eine erste Komponente 6a ist eine poröse Folie, die einen Abschnitt der Oberseite des Stechelements 1 bedeckt und in dem bedeckten Abschnitt den Kapillarkanal 3 blockiert, indem sie ihn ausfüllt. Zwischen dem Sterilschutz und dem Stechelement 1 im Bereich des ausgefüllten Kapillarkanals 3 bleibt allenfalls ein kleiner Spalt von weniger als 10 µm. Die zweite Komponente 6b ist bei dem dargestellten Ausführungsbeispiel ebenfalls eine Folie, beispielsweise aus Papier, Metall oder -was bevorzugt ist - aus einem Polymer.

Bei der Herstellung wird das Stechelement 1 zwischen die beiden Folien 6a, 6b gelegt und verpresst. Wegen ihrer Porosität lässt sich die erste Folie 6a dabei stark komprimieren. Die erste Folie 6a wird beim Verpressen von dem Stechelement 1 auf beiden Seiten des Kapillarkanals 3 stark komprimiert. Im Bereich des Kapillarkanals 3 wird die Folie 6a weniger stark oder gar nicht komprimiert, so dass der Kapillarkanal 3 von der Folie 6a ausgefüllt wird. Bevorzugt wird das Verpressen bei einer erhöhten Temperatur durchgeführt, beispielsweise bei mehr als 80°C, insbesondere mehr als 100°C, damit sich die erste Folie 6a besser verformen und den Kapillarkanal 3 ausfüllen kann.

Nach dem Verpressen werden die beiden Folien 6a, 6b miteinander verbunden, bevorzugt stoffschlüssig, beispielsweise durch Kleben oder Schweißen.

Die poröse Folie 6a ist bevorzugt dicker als das Stechelement 1, insbesondere ist die Dicke der Folie 6a größer als die Tiefe des Kapillarkanals 3. Bei dem dargestellten Ausführungsbeispiel ist die erste Folie 6a mehr als zehnmal, bevorzugt mehr als zwanzigmal, so dick wie die zweite Folie 6b. Die erste Folie 6a hat bei dem dargestellten Ausführungsbeispiel eine Dicke von mehr als 100 µm, beispielsweise 130 µm. Die zweite Folie eine Dicke von weniger als 10 µm, beispielsweise 6 µm.

Die erste Folie 6a kann beispielsweise eine mikroporöse Membran sein, wie sie beispielsweise von der Firma Pall, Dreieich, Deutschland, unter der Bezeichnung BTS 25 vertrieben wird. Die erste Folie 6a kann beispielsweise auch aus einem Schaumstoff, beispielsweise aus Polystyrol, sein. Dabei sind geschlossenzellige Schaumstoffe bevorzugt, aber nicht zwingend erforderlich. Beispielsweise kann die erste Folie 6a auf ihrer von dem Stechelement 1 abgewandten Seite mit einer Siegelfolie bedeckt werden. Die Siegelfolie kann aufgeklebt oder aufgeschweißt werden. Bevorzugt bedeckt eine solche Siegelfolie die gesamte Oberseite der ersten Folie 6a. Die Siegelfolie kann seitlich über die erste Folie 6a herausragen und mit der zweiten Folie 6b verklebt oder verschweißt sein.

Als Material für die erste Komponente 6a des Sterilschutzes sind Polymermaterialien bevorzugt, die leicht verformbar sind, um einen Abschnitt der Kapillarstruktur möglichst vollständig auszufüllen. Ferner sollte sich das Material möglichst leicht wieder aus der Kapillarstruktur entfernen lassen und hydrophile Eigenschaften der Kapillarstruktur möglichst nicht beeinträchtigen.

Nach dem Anbringen des Sterilschutzes 6a, 6b wird das Stechelement 1 durch Strahlungseinwirkungen sterilisiert, beispielsweise durch Gammastrahlung. Eine Strahlungsdosis von 25 kGray reicht in der Regel aus. Das sterilisierte Stechelement 1 lässt sich anschließend ohne Schwierigkeiten handhaben, da die bei einem Stich in den Körper eines Patienten eindringenden Teile durch den Sterilschutz 6a, 6b geschützt sind. Das sterilisierte Stechelement 1 wird an dem Trägerkörper 2 befestigt, der einen Testbereich 5 mit Nachweisreagenzien zur Bestimmung einer Analytkonzentration einer Körperflüssigkeitsprobe trägt. Beispielsweise kann das Stechelement 1 mit dem Trägerkörper 2 verklebt und/oder in eine passende Ausnehmung des Trägerkörpers 2 gesteckt werden.

Auf die von dem Stechelement 1 abgewandte Oberfläche der ersten Folie 6a und bevorzugt auch auf den Trägerkörper 2 wird eine Folie 8 aufgeklebt. Bevorzugt handelt es sich bei der Folie 8 um ein doppelseitiges Klebeband, mit dem die Lanzette anschließend auf ein Trägerband 7 geklebt wird. Dabei ist nicht erforderlich das die Folie 8 die gesamte Oberfläche der porösen Folie 6a oder des Trägerkörpers 2 bedeckt. Es genügt, wenn die Folie 8 einen Teil der Oberfläche des Trägerkörpers 2 oder der porösen Folie 6a bedeckt.

In Figur 4 ist eine Lanzette mit Sterilschutz 6a, 6b und Klebefolie 8 in einer Seitenansicht dargestellt. Zum Entfernen des Sterilschutzes kann das Trägerband 7 gebogen werden, wie es in Figur 5 schematisch dargestellt ist. Durch die Biegung des Trägerbandes 7 klappt der Sterilschutz 6a, 6b von dem Stechelement 1 weg. Dabei durchbricht das Stechelement 1 die zweite Folie 6b, so dass diese zusammen mit der ersten Folie 6a weggeklappt wird. Einzelheiten zu einem Handgerät mit einer Einrichtung zum Biegen eines Trägerbandes sind beispielsweise aus der WO 2008/083844 A1 bekannt.

Das Trägerband 7 ist bevorzugt eine Polymerfolie, deren Dicker größer als die Dicke der zweiten Folie 6b des Sterilschutzes ist, beispielsweise mindestens um die Hälfte dicker, insbesondere mindestens doppelt so dick sein. Beispielsweise kann das Trägerband 7 eine Polyestherfolie sein und eine Dicke von beispielsweise 12 µm aufweisen.

### Bezugszahlen

- 1: Stechelement
- 2: Trägerkörper
- 3: Kapillarkanal
- 4: Spitze des Stechelements
- 5: Testbereich
- 6: Sterilschutz
- 6a: erste Komponente des Sterilschutzes
- 6b: zweite Komponente des Sterilschutzes
- 7: Trägerband
- 8: Klebefolie

## Patentansprüche

1. Lanzette mit
einem Stechelement (1) zum Einstich in die Haut eines Patienten,
einem Testbereich (5) mit Nachweisreagenzien zur Bestimmung einer Analytkonzentration einer Körperflüssigkeitsprobe,
einer in dem Stechelement (1) angeordneten Kapillarstruktur (3), um eine Körperflüssigkeitsprobe zu dem Testbereich (5) zu transportieren, und
einem Sterilschutz (6), der die Spitze des Stechelements (4) umschließt, **dadurch gekennzeichnet, dass**
die Kapillarstruktur (3) in einem von dem Sterilschutz (6) umgebenen Abschnitt blockiert ist.

2. Lanzette nach Anspruch 1, **dadurch gekennzeichnet, dass** die Kapillarstruktur (3) blockiert ist, indem der Sterilschutz (6) die Kapillarstruktur (3) zumindest auf einem Teil ihrer Länge ausfüllt.

3. Lanzette nach einem der vorstehenden Ansprüche, **dadurch gekennzeichnet, dass** die Lanzette aus dem Sterilschutz (6) herausragt.

4. Lanzette nach einem der vorstehenden Ansprüche, **dadurch gekennzeichnet, dass** Sterilschutz (6) aus mindestens zwei Komponenten zusammen gefügt ist, wobei eine erste Komponente (6a) einen Abschnitt einer Oberseite des Stechelements (1) bedeckt und in dem bedeckten Abschnitt den Kapillarkanal (3) ausfüllt, und eine zweite Komponente (6b) einen Abschnitt einer Unterseite des Stechelements (1) bedeckt.

5. Lanzette nach Anspruch 4, **dadurch gekennzeichnet, dass** mindestens eine der beiden Komponenten (6a, 6b) eine Folie (8) ist.

6. Lanzette nach einem der Ansprüche 3 bis 5, **dadurch gekennzeichnet, dass** die erste Komponente (6a) porös ist.

7. Lanzette nach Anspruch 6, **dadurch gekennzeichnet, dass** die erste Komponente (6a) ein Polymerschaum ist.

8. Lanzette nach einem der Ansprüche 3 bis 7, **dadurch gekennzeichnet, dass** auf die von dem Stechelement (1) abgewandte Seite der ersten Komponente (6a) eine Folie (8) geklebt ist.

9. Lanzette nach Anspruch 8, **dadurch gekennzeichnet, dass** die auf der ersten Komponente (6a) geklebte Folie (8) auch auf einen aus dem Sterilschutz (6) herausragenden Teil der Lanzette geklebt ist.

10. Lanzette nach einem der Ansprüche 3 bis 9, **dadurch gekennzeichnet, dass** die erste Komponente (6a) dicker als die zweite Komponente (6b) ist, vorzugsweise mindestens fünfmal so dick ist, insbesondere mindestens zehnmal so dick ist.

11. Lanzette nach einem der vorstehenden Ansprüche, **dadurch gekennzeichnet, dass** zumindest ein Abschnitt der Kapillarstruktur (3) ein Kapillarkanal, vorzugsweise in Form einer Rinne, ist.

12. Lanzette nach einem der vorstehenden Ansprüche, **dadurch gekennzeichnet, dass** das Stechelement (1) an einem Trägerkörper (2) befestigt ist, der den Testbereich (5) trägt.

13. Trägerband mit mehreren darauf angeordneten Lanzetten, **dadurch gekennzeichnet, dass** die Lanzetten nach einem der vorstehenden Ansprüche ausgebildet sind.

14. Verfahren zum Herstellen einer Lanzette mit den folgenden Schritten:
a) Herstellen eines Stechelements (1), das auf seiner Oberseite einen nach oben offenen Kapillarkanal (3) aufweist,
b) Bedecken eines Abschnitts der Oberseite des Stechelements (1) und Blockieren der Kapillarstruktur (3) in diesem Abschnitt mit einem ersten Material (6a),
c) Bedecken eines Abschnitts der Unterseite mit einem zweiten Material (6b),
d) Sterilisieren des Stechelements (1),
e) Anbringen eines Testbereichs (5) mit Nachweisreagenzien an dem Stechelement (1).

15. Verfahren nach Anspruch 14, **dadurch gekennzeichnet, dass** das erste Material (6a) in die Kapillarstruktur (3) hinein gepresst wird.
